# EUROPEAN PATENT SPECIFICATION

(11) **EP 0 976 757 B1**
(45) Date of publication and mention of the grant of the patent: **01.06.2005**
(21) Application number: 99850103.5
(22) Date of filing: 09.06.1999
(51) Int. Cl.: C07H 23/00

(54) **A method of purifying vitamin B12 and/or derivatives thereof**
Verfahren zur Reinigung von Vitamin B12 und/oder deren Derivate
Méthode de purification de la vitamine B12 et/ou de ses dérivés

(30) Priority: 11.06.1998 JP 16397698
(43) Date of publication of application: 02.02.2000
(73) Proprietor: Nippon Mitsubishi Oil Corporation, Tokyo (JP)
(72) Inventor: Hirayama, Takayuki, c/o Nippon Mitsubishi Oil Corp, Yokohama-shi, Kanagawa-ken (JP); Noboru, Endo, c/o Nippon Mitsubishi Oil Corp., Yokohama-shi, Kanagawa-ken (JP); Mizuta, Haruyoshi, c/o Nippon Mitsubishi Oil Corp., Yokohama-shi, Kanagawa-ken (JP)
(74) Representative: Avellan-Hultman, Olle

(56) References cited:
- EP-A- 0 109 859
- GB-A- 718 454
- GB-A- 816 426
- GB-A- 833 067

## Description

This invention relates to a method of purifying vitamin B₁₂ and/or derivatives thereof.

Recently, vitamin B₁₂ and derivatives thereof have been mass-produced by a method such as synthesis and extraction and placed on the market. Upon purification of crude vitamin B₁₂ and vitamin B₁₂ derivatives, such as methylcobalamine, hydroxocobalamine and coenzyme-type vitamin B₁₂, each of which is produced by a variety of methods, recrystallization is generally conducted at the final stage of purification.

Vitamin B₁₂ and derivatives thereof are highly soluble in water. Therefore, cooling crystallization method using pure water as a solvent poses problems of poor recovery, resulting in high production cost.

Theoretically, it is possible to recover reasonable amounts of vitamin B₁₂ and derivatives thereof from an aqueous solution containing crude vitamin B₁₂ and derivatives thereof by heating the solution to increase the concentration thereof. However, in fact, problems arise that in the last stages of the concentration, i.e. where the concentration of the solution is increased, decomposition occurs due to heating or the viscosity of the aqueous solution becomes too high, resulting in that the operations such as charging into the crystallization bath and filtration become tedious.

For the reason of these problems, cooling-crystallization or reprecipitation has been employed generally using water in combination with hydrophilic organic solvents, such as methanol, acetone or dioxane. Such methods using methanol are disclosed in Japanese Patent Publication Nos. 46-8862 and 46-43389, while those using acetone are disclosed in Japanese Patent Publication Nos. 45-38059, 46-14664, 47-34720, 50-38120, 51-120 and 64-16597.

However, when using an organic solvent, it possibly mixes into the resulting crystals and then adversely affects the human body. In order to avoid such risk, it has been proposed to remove the organic solvent from the crystals. For example, Japanese Patent Laid-Open Publication No. 49-18896 discloses a method of reducing the content of acetone to 0.1 %, in which method the crystals of coenzyme-type vitamin B₁₂ containing acetone are charged into an airtight vessel saturated with steam and left standing at room temperature or at an elevated temperature for a period ranging from a few hours to a few days or exposed to ventilation with steam-saturated air.

However, even though such a measure is taken, it cannot, in practice, completely remove the organic solvent from the crystals and causes an increase in production costs.

In view of the foregoing existing circumstances with which the prior art techniques are confronted, it has been found after extensive research that vitamin B₁₂ and derivatives thereof can be purified with increased recoveries merely using water as a solvent and without using any organic solvent.

According to the present invention, there is provided a method of purifying vitamin B₁₂, methylcobalamine, hydroxocobalamine, or coenzyme-type vitamin B₁₂ which comprises the steps of heating an aqueous solution containing vitamin B₁₂, methylcobalamine, hydroxocobalamine, or coenzyme-type vitamin B₁₂, in an amount of 50-300g/L at a temperature of 50-80°C; adding thereto a water soluble ionic compound selected from the group consisting of sodium phthalate, sodium acetate, potassium acetate, sodium chloride, and calcium butyrate in an amount of 20 to 70 mass % based on the mass of said solution provided that in the case where the solution is strongly alkalized after the addition of said ionic compound, it is maintained around neutral by adding an acid selected from the group consisting of hydrochloric acid, sulfuric acid, acetic acid, oxalic acid, and formic acid; and cooling the solution to a temperature of 5-25°C such that vitamin B₁₂, methylcobalamine, hydroxocobalamine, or coenzyme-type vitamin B₁₂ is crystallized.

The present invention will be further described in detail hereinbelow.

Vitamin B₁₂, i.e., cyanocobalamin is usually obtained by the cyanogenation of coenzyme-type vitamin B₁₂ produced by fermentation using microorganism producing coenzyme-type vitamin B₁₂. The inventive method is, however, applicable to the purification of vitamin B₁₂ obtained by any other method.

The term "vitamin B₁₂ derivative" referred to herein includes methylcobalamin, hydroxocobalamin and coenzyme-type vitamin B₁₂ and so on.

Coenzyme-type vitamin B₁₂ is industrially produced by utilizing chemical synthesis from a starting material such as cyanocobalamin or hydroxocobalamin. When cyanocobalamin is used as the starting material, it may be subjected to a reaction with 5'- tosyladenosine, as disclosed in Japanese Patent Publication Nos. 45-38058 and 52-24040. Furthermore, when hydroxocobalamin is used as the starting material, a synthesizing method may be employed in which it is reacted with 5'-deoxy-5'-halogenoadenosine, as disclosed in Japanese Patent Publication Nos. 45-6273 and 46-29154, and/or with 5'-tosyladenosine, as disclosed in Japanese Patent No. 52-24040.

In the commercial production of hydroxocobalamin, it may be chemically synthesized using cyanocobalamin as a starting material or may be produced by fermentation. In the former method, the starting material is brought into contact with zinc (Japanese Patent Publication Nos. 39-18418 and 46-14664) or with a Pd catalyst. The latter method is disclosed in Japanese Laid-Open Patent Publication No. 5-86089.

As a production method for methylcobalamin, there may be exemplified those disclosed in Japanese Laid-Open Patent Publication Nos. 47-34720 and 57-27119, in which cyanocobalamin is used as a starting material. Alternatively, as disclosed in Japanese Laid-Open Patent Publication Nos. 47-34720, 50-38120 and 51-120, a method may be employed of chemically synthesizing methylcobalamin by reacting hydroxocobalamin used as a starting material with a reducing agent, such as zinc or CH₃MgBr, or with an oxalic monomethyl ester.

When an aqueous solution of vitamin B₁₂ and/or a derivative thereof further containing an organic solvent is used for the inventive method, it is preferred to reduce the amount of the organic solvent beforehand to less than 1,000 ppm by way of distillation or membrane-separation. If vitamin B₁₂ and/or a derivative thereof in the solution is extremely low in concentration, it is also preferred to concentrate the solution beforehand. It is preferred to adjust the concentration of vitamin B₁₂ and/or a derivative thereof to 10 - 500 grams, preferably 50 - 300 grams per liter of the aqueous solution.

According to the present invention, a solution of vitamin B₁₂ and/or a derivative thereof is heated to generally 40 - 90 °C, preferably 50 - 80 °C and mixed with a water soluble ionic compound. Although not restricted, the water soluble ionic compound is added selectively in an amount ranging from generally 10 to 80 mass percent, preferably 20 to 70 mass percent by, based on the mass of the solution. When the amount of the water soluble ionic compound is selected, it is preferred to estimate the possible maximum amount beforehand, so that the compound does not precipitate at the lowest temperature which is finally reached upon crystallization.

If the solution is strongly alkalinized by the addition of the ionic compound, it is preferred to add acids, such as hydrochloric acid, sulfuric acid, acetic acid, oxalic acid and formic acid to keep the pH of the solution around neutrality.

The solution mixed in which the ionic compounds are dissolved is then gradually cooled down to a temperature of 0 to 30 °C, preferably 5 to 25 °C, whereby vitamin B₁₂ and/or derivatives thereof are crystallized.

Seed crystals may be added to the solution prior to the crystallization, so as to unify the particle diameter of the resulting crystals.

The precipitated crystals resulting from cooling are preferably suction-filtered and then immediately washed with cold water before the water on the crystals dries out. The crystals thus obtained are placed on the market after being subjected to vacuum drying.

The inventive method can produce vitamin B₁₂ or derivatives thereof which do not contain any organic solvent therein and can increase the recoveries significantly compared to a method using only water as a solvent, thus resulting in a great value to the industry.

The invention will be further described by way of the following examples, which are provided for illustrative purposes only.

### Example 1

A 930 ml solution containing 15.5 grams vitamin B₁₂ was heated to a temperature of 60 °C and mixed with 740 grams of sodium phthalate hemihydrate with stirring. While the stirring was continued, 1N hydrochloric acid was added to the solution to adjust the pH to 7. The solution was slowly cooled down to a temperature of 10 °C over 10 hours with stirring and thereafter left standing at a temperature of 10 °C for 3 hours. The crystals thus precipitated were subjected to vacuum filtration with a 10 micron mesh filter and then washed with 30 ml of cold water having a temperature of 8 °C, followed by 10 hour of vacuum drying at a temperature of 40 °C. The recovery was 89%. The purity of the resulting product was examined in accordance with the Japanese Pharmacopoeia, and the result thereof was 97% which fulfilled the criterion.

### Comparative Example 1

Vitamin B₁₂ was recovered by following the procedure of Example 1 except omitting the addition of sodium phthalate and 1N hydrochloric acid. The recovery was only 36%.

### Example 2

620 ml solution containing 13.0 grams coenzyme type vitamin B₁₂ was heated to 60 °C and then gradually mixed with 540 grams sodium acetate trihydrate with stirring. While stirring was continued, acetic acid was added to the solution to adjust the pH to 7. The solution was slowly cooled down to 10 °C over 10 hours with stirring and then left standing at a temperature of 10 °C for 3 hours. The crystals thus precipitated were subjected to vacuum filtration with a 10 micron mesh filter and immediately thereafter washed with 30 ml of cold water having a temperature of 8 °C, followed by 10 hour of vacuum drying at a temperature of 40 °C. The recovery was 73%. The resulting product was subjected to cyanogenation to examine the purity. The result was 96%.

### Comparative Example 2

Coenzyme-type vitamin B₁₂ was recovered by following the procedure of Example 2 except omitting the addition of sodium acetate and acetic acid. The recovery was only 25%.

### Example 3

A 310 ml solution containing 12.5 grams hydroxocobalamin was heated to 60 °C and then gradually mixed with 75 grams of sodium chloride with stirring. While stirring was continued, acetic acid was added to the solution to adjust the pH to 6. The solution was cooled down to 10 °C over 10 hours with stirring and then left standing for 3 hours. The crystals thus precipitated were subjected to vacuum filtration with a 10 micron mesh filter and immediately thereafter washed with 30 ml of cold water having a temperature of 8 °C, followed by 10 hour of vacuum drying at a temperature of 40 °C. The recovery was 71%. The acetic acid stability test in accordance with the Japanese Pharmacopoeia was conducted and revealed the presence of acetic acid. The purity of the resulting product was also examined in accordance with the Japanese Pharmacopoeia, and the result was 99% which fulfilled the criterion.

### Comparative Example 3

The recovery of hydroxocobalamin was conducted by following the procedure of Example 3 except omitting the addition of sodium chloride. No crystals were recovered at all.

### Example 4

A 690 ml of the solution containing 11.0 grams methylcobalamin was heated to 60 °C and mixed with 122 grams of calcium butyrate dihydrate with stirring. The solution was cooled down to a temperature of 10 °C over 10 hours while being stirred and thereafter left standing at a temperature of 10 °C for 3 hours. The crystals thus precipitated were subjected to vacuum filtration with a 10 micron mesh filter and then washed with 30 ml of cold water having a temperature of 8 °C, followed by 10 hour of vacuum drying at a temperature of 40 °C. The recovery was 91%. The resulting product was subjected to cyanogenation to examine the purity. The result was 97%.

### Comparative Example 4

Methylcobalamin was recovered by following the procedure of Example 4 except omitting the addition of calcium butyrate. The recovery was only 41%.

## Claims

1. A method of purifying vitamin B₁₂, methylcobalamine, hydroxocobalamine, or coenzyme-type vitamin B₁₂ which comprises the steps of heating an aqueous solution containing vitamin B₁₂, methylcobalamine, hydroxocobalamine, or coenzyme-type vitamin B₁₂, in an amount of 50-300g/L at a temperature of 50-80°C; adding thereto a water soluble ionic compound selected from the group consisting of sodium phthalate, sodium acetate, potassium acetate, sodium chloride, and calcium butyrate in an amount of 20 to 70 mass % based on the mass of said solution provided that in the case where the solution is strongly alkalized after the addition of said ionic compound, it is maintained around neutral by adding an acid selected from the group consisting of hydrochloric acid, sulfuric acid, acetic acid, oxalic acid, and formic acid; and cooling the solution to a temperature of 5-25°C such that vitamin B₁₂, methylcobalamine, hydroxocobalamine, or coenzyme-type vitamin B₁₂ is crystallized.

## Patentansprüche

1. Verfahren zur Reinigung von Vitamin B₁₂, Methylcobalamin, Hydroxocobalamin oder Vitamin B₁₂ vom Coenzym-Typ, welches die Schritte umfasst: Erwärmen einer wässrigen Lösung, die Vitamin B₁₂, Methylcobalamin, Hydroxocobalamin oder Vitamin B₁₂ vom Coenzym-Typ in einer Menge von 50 - 300 g/l enthält, bei einer Temperatur von 50 - 80 °C; Dazugeben einer wasserlöslichen ionischen Verbindung, die aus der Gruppe ausgewählt ist, die aus Natriumphthalat, Natriumacetat, Kaliumacetat, Natriumchlorid und Calciumbutyrat besteht, in einer Menge von 20 bis 70 Massenprozent, bezogen auf die Masse der Lösung, mit der Maßgabe, dass in dem Fall, in dem die Lösung nach der Zugabe der ionischen Verbindung stark alkalisiert ist, diese durch Zugabe einer Säure, die aus der Gruppe ausgewählt ist, die aus Chlorwasserstoffsäure, Schwefelsäure, Essigsäure, Oxalsäure und Ameisensäure besteht, bei etwa neutral gehalten wird; und Abkühlen der Lösung auf eine Temperatur von 5 - 25 °C, so dass Vitamin B₁₂, Methylcobalamin, Hydroxocobalamin oder Vitamin B₁₂ vom Coenzym-Typ kristallisiert.

## Revendications

1. Méthode de purification de la vitamine B₁₂, de la méthylcobalamine, de l'hydroxocobalamine, ou de la vitamine B₁₂ du type coenzyme qui comprend les étapes consistant à chauffer une solution aqueuse contenant de la vitamine B₁₂, de la méthylcobalamine, de l'hydroxocobalamine ou de la vitamine B₁₂ du type coenzyme, en une quantité allant de 50 à 300 g/l à une température allant de 50 à 80 °C ; à y ajouter un composé ionique hydrosoluble sélectionné dans le groupe constitué par le phtalate de sodium, l'acétate de sodium, l'acétate de potassium, le chlorure de sodium et le butyrate de calcium en une quantité allant de 20 à 70 % en masse en se basant sur la masse de ladite solution, à condition que dans le cas où la solution est fortement alcalinisée après l'ajout dudit composé ionique, elle soit conservée à peu près neutre en ajoutant un acide sélectionné dans le groupe constitué par l'acide chlorhydrique, l'acide sulfurique, l'acide acétique, l'acide oxalique et l'acide formique ; et à refroidir la solution à une température allant de 5 à 25 °C, de telle sorte que la vitamine B₁₂, la méthylcobalamine, l'hydroxocobalamine ou la vitamine B₁₂ du type coenzyme soit cristallisée.
